# EUROPEAN PATENT SPECIFICATION

(11) **EP 2 098 180 B1**
(45) Date of publication and mention of the grant of the patent: **13.06.2012**
(21) Application number: 09006385.0
(22) Date of filing: 13.03.2006
(51) Int. Cl.: A61B 17/34, A61B 17/00, A61M 25/06, A61B 19/00

(54) **Radially expandable access system including trocar seal**
Radial ausdehnbares Zugangssystem mit Trokarverschluss
Système d'accès extensible radialement incluant un joint de trocart

(30) Priority: 16.03.2005 US 81766
(43) Date of publication of application: 09.09.2009
(62) Divisional of application: 06005056.4
(73) Proprietor: Tyco Healthcare Group LP, Mansfield, MA 02048 (US)
(72) Inventor: Farascioni, David, Bethel, CT 06801 (US)
(74) Representative: Soames, Candida Jane

(56) References cited:
- WO-A-03/071926
- US-A- 5 797 888
- US-A1- 2004 199 121

## Description

### BACKGROUND

### Technical Field

The present invention relates generally to apparatus for providing access to an internal operative site during a surgical procedure and, more particularly, to access systems which may be percutaneously or otherwise introduced while in a narrow diameter configuration and which after introduction may be radially expanded to accommodate passage of larger diameter surgical instruments therethrough.

### Background of related Art

Minimally invasive surgical procedures rely on obtaining percutaneous access to an internal surgical site using small-diameter access tubes (typically 5 to 12 mm), usually referred to as trocars, which penetrate through the skin and which open to the desired surgical site. A viewing scope is then introduced through one such trocar, and the surgeon operates using instruments introduced through other appropriately placed trocars while viewing the operative site on a video monitor connected to the viewing scope. The surgeon is thus able to perform a wide variety of surgical procedures requiring only several 5mm to 12 mm punctures at the surgical site. As a result, patient trauma and recovery time are typically reduced.

Particular minimally invasive surgical procedures are often referred to based on the type of scope used to view the region of the body which is the operative site. For example, procedures in the abdominal area, which rely on a laparoscope for viewing, are typically referred to as laparoscopic procedures. In such laparoscopic procedures, the patient's abdominal region is typically insufflated (filled with pressured gas) to raise the abdominal wall and create sufficient operating space to perform a desired procedure. The trocars used in laparoscopic procedures must therefore include a valve at their proximal end to allow passage of the scope or surgical instruments while inhibiting leakage of the insufflating gas. It has also been proposed to perform laparoscopic procedures by mechanically expanding the abdomen rather than using insufflation.

Recently, a radially expandable access system has been developed, as shown and described in U.S. Pat. Nos. 5,183,464; 5,431,676; 5,814,058; 5,927,319; 6,080,174; 6,245,052; 6,325,812; 6,494,893; and 6,589,225, as well as in U.S. Pat. Appl. Nos. 2001/0039430; 2002/0002360; 2003/0023259; and 2003/0199809.
The radially expandable access systems disclosed therein may include a pneumoperitoneum needle, an expandable sleeve component which is percutaneously introduced while positioned over the pneumopentoneum needle, a cannula having a pneumostasis valve permanently affixed at its proximal end, and an obturator which is removably inserted into the cannula to form an expansion member for the sleeve. After the needle/sleeve assembly has been percutaneously introduced, and the peritoneal cavity insufflated in the case of laparoscopic procedures, the needle is removed from the sleeve, and the cannala/obturator assembly introduced through the sleeve. The sleeve, which initially has a diameter in the range of 2-3 mm, is thus expanded to a final diameter depending on the cannula size, which can be selected from 5 mm, 10 mm, or 12 mm. Use of the radially expandable access system has many advantages, including reduced trauma to the patient and the ability to replace a cannula with a larger diameter cannula through a previously introduced sleeve.

US 5797888 (A) discloses a cannula for insertion through an anatomical cavity wall to establish communication with the anatomical cavity, which includes an elongate tubular body having a distal end adapted to be disposed within the anatomical cavity and a proximal end adapted to be disposed externally of the anatomical cavity, a seal including a seal member disposed along the tubular body of the cannula, and a tubular pusher disposed in the tubular body and insertable through the seal to move the seal member from a normally closed position preventing fluid flow through the cannula to an open position allowing instruments of various sizes to be introduced through the tubular body via the tubular pusher without contacting the seal.

The preamble of claim 1 is based as this document.

US 2004/0199121 (A1) discloses an access system for forming and enlarging a percutaneous penetration. The apparatus includes a radially expandable dilation assembly having a radially expandable sleeve body and a handle portion operatively coupled to the proximal end of the radially expandable sleeve body. The access system further includes an expansion assembly having an expansion member configured and adapted to radially expand the sleeve body upon insertion therein of the expandable member. The handle portion including a seal disposed across an aperture formed therein for creating a fluid-tight seal around a proximal end of the expansion member. The handle portion including a valve stem operatively and fluidly coupled thereto for delivering a fluid into the aperture of the handle portion. The expansion member including means for reducing the force required to slidably insert the expansion member into the sleeve body of the dilation assembly.

While the radially expandable access system represents a substantial advance over conventional trocars, the need and desire exists for improved radially expandable access systems, component kits for such systems, and methods for reconstructing and reusing such systems.

### SUMMARY

The present disclosure relates to access systems which may be percutaneously or otherwise introduced while in a narrow diameter configuration and, which after introduction, may be radially expanded to accommodate passage of larger diameter surgical instruments therethrough.

According to the present invention, an access system is provided. The access system includes a radially expandable sleeve component including a handle having a passage therethrough; and a sleeve body having a proximal end connected to the handle, a distal end, and an axial lumen aligned with the passage of the handle, the sleeve body having a length The distal end of the sleeve body is flared radially outward. The access system further includes a cannula tube having a proximal end, a distal end, and a lumen extending therethrough. The cannula tube is sized for reception in the aperture of the handle of the radially expandable sleeve components The cannula tube has a length which is shorter than the length of the sleeve body when the cannula tube is fully inserted into the sleeve body of the radially expendable sleeve component. Further features of the present invention are defined in claim 1.

Desirably, when the cannula tube is fully inserted into the sleeve body of the radially expandable sleeve component the flared distal end of the sleeve body extends beyond the distal end of the cannula tube. The radially expandable sleeve further includes a sheath encasing the sleeve body along at least a portion of the length thereof.

The sleeve body may be constructed from a radially expandable braid. The braid may be formed of a mesh of non-elastic filaments which axially shortens the length of the sleeve body as the sleeve body is radially expanded

Desirably, the sheath maintains the flared distal end of sleeve body in a radially unexpanded condition. It is contemplated that the flared distal end of the sleeve body takes form upon removal of the sheath therefrom.

The access system may further include an obturator removably receivable in the lumen of the cannula tube. The obturator has a tapered distal end which extends distally from the distal end of the cannula tube when the obturator is disposed in the lumen of the cannula tube. The access system may further include a pneumoperitoneum needle including a tubular needle; and an internal stylet removably receivable within the tubular needle.

Other objects and features of the present disclosure will become apparent from consideration of the following description taken in conjunction with the accompanying drawings.

### BRIEF DESCRIPTION OF THE DRAWING

By way of example only, embodiments of the radially expandable access system of the present disclosure, will be described with reference to the accompanying drawings, in which

FIG. 1 is a side view of a radially expandable sleeve component of the access system according to the present disclosure, including a removable sheath encasing a tubular braid portion thereof,

FIG. 1A is a longitudinal cross-sectional view of the radially expandable sleeve component of FIG. 1;

FIG. 2 is a side view of the radially expandable sleeve component of FIG. 1 with the sheath removed from the tubular braid portion thereof;

FIG. 3 is a side view of a prior art pneumoperitoneum needle component for use with the radially expandable sleeve component of FIGS. 1 and 2;

FIG. 4 is a side view of a prior art cannula assembly for use with the radially expandable sleeve component of FIGS. 1 and 2, shown with the cannula body, cannula hub, and valve cap removed or separated from each other, and further shown with the valve cap in partial section;

FIG. 5 is a side view of a prior art obturator component for use with the radially expandable sleeve component of FIGS. 1 and 2, and cannula assembly of FIG. 3;

FIG. 6 is a side view of the radially expandable sleeve component of FIGS. **1** and 2 having the cannula assembly of FIG. 3 operatively associated therewith and with the valve cap of the cannula assembly and the handle of the radially expandable sleeve component shown in partial section;

FIG. 7 is a side view of the radially expandable sleeve component of FIGS. 1 and 2 having the cannula assembly of FIG. 3 operatively associated therewith and a surgical instrument extending therethrough, with the valve cap of the cannula assembly and the handle of the radially expandable sleeve component being shown in partial section;

FIG. 7A is a cross-sectional side view of the radially expandable sleeve component of FIGS. 1 and 2 having the cannula assembly of FIG. 3 operatively associated therewith and a surgical instrument extending therethrough; and

FIGS. 8-13 illustrate use of the radially expandable sleeve component of FIGS. 1 and 2 in providing access to a patient's abdomen.

### DETAILED DESCRIPTION OF PREFERRED EMBODIMENTS

The access system of the present disclosure is useful for forming and enlarging percutaneous penetrations into a variety of target locations within a patient's body for a multiplicity of purposes. Such purposes include drainage, intra-organ drug administration, feeding, perfusion, aspiration, and the like, most usually being the introduction of viewing scopes and surgical instruments for use in minimally invasive surgical procedures, such as laparoscopic procedures, thoracosoopic procedures, arthrosoopic procedures, endoscopic procedures, and the like. In addition to percutaneous procedures, the access system of the present disclosure will find use in hysteroscopic, colonoscopic, and other procedures where access is established through existing body orifices.

The access systems of the present disclosure are particularly valuable in percutaneous procedures since they will create a very small initial penetration, usually being below about 5 mm, more usually being below about 4 mm, frequently being below about 3.5 mm, and preferably being 3 mm or below. The penetration will be subsequently enlarged to a desired final size, usually having a final diameter in the range from about 5 mm to 15 mm, more usually being from about 5 mm to 12 mm, and typically being from about 5 mm to 10 mm. The enlarged penetration will define an access lumen from the outside of the patient's body to the desired internal location, and it is a particular advantage of the present disclosure that the diameter of the access lumen may be changed as will be described in more detail hereinafter. In non-percutaneous procedures, the access system is valuable since it is capable of passing through the existing body orifice in its narrow-diameter configuration and be subsequently expanded with minimum discomfort and trauma to the patient.

The access system of the present disclosure includes a number of individual components that may be assembled into different size configurations. The assembled components may also be disassembled after use; and the components selectively sterilized or replaced prior to reassembling the access system for further use with a different patient. The different components and component assemblies and subassemblies will be described in greater detail below.

Sterilization of the components of the trocar system disclosed herein may be accomplished by any suitable conventional sterilization technique, including heat, e.g., steam and autoclaving, chemical treatment, e.g., ethylene oxide exposure; radiation, and the like. After use, reusable components will be washed to remove blood and other contaminating substances and then sterilized, preferably by exposure to steam. Disposable components will usually be radiation sterilized in their packages prior to distribution. Thus, disposable components will usually be ready to use out of the package.

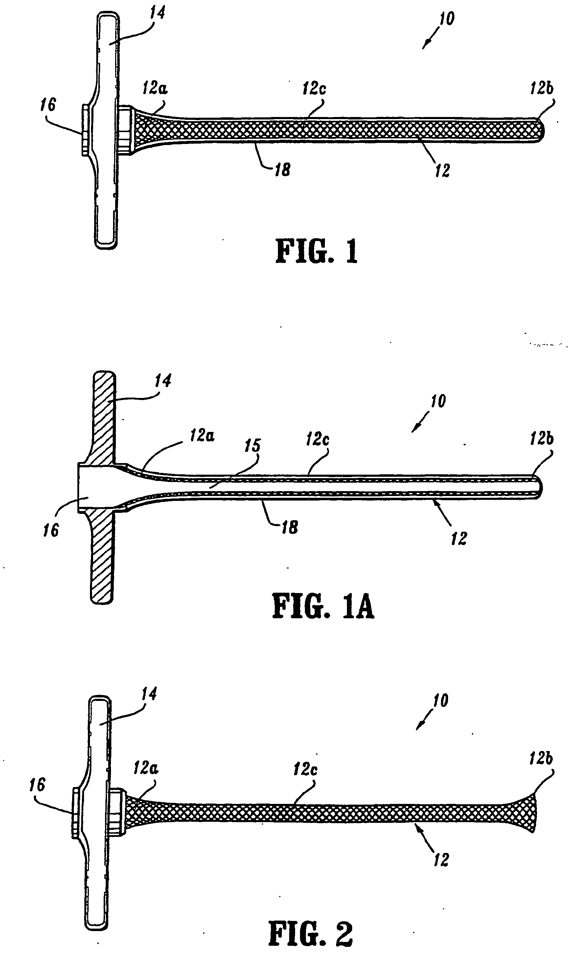

Referring initially to FIGS. 1, 1A and 2, wherein like reference numerals identify similar or identical structural elements, a radially expandable sleeve component or trocar seal, according to an embodiment of the present disclosure, for use as part of an access system, is generally designated as 10. As used herein, the term "distal" refers to that portion of the tool, or component thereof which is further from the user while the term "proximal" refers to that portion of the tool or component thereof which is closer to the user.

As seen in FIGS. 1, 1A and 2, sleeve component 10 includes a sleeve body 12 defining a lumen 15 (see FIG. 1A) from a proximal end 12a to a distal end 12b thereof, and a handle 14 operatively connected to proximal end 12a of sleeve body 12. Preferably, sleeve body 12 is constructed from a radially expandable braid, desirably inelastic, having an inner diameter of about 2 mm and an outer diameter of about 3.5 mm. Handle 14 includes a passage 16 (see FIG. 1A) formed therethrough, which passage 16 is substantially aligned with the lumen of sleeve body 12. Desirably and typically a connector (not shown) is provided about passage 16 for selectively engaging a complementary connector provided on a cannula assembly 40. For example, the complementary connectors may take the form of threads, bayonet fittings, and the like. As will be described in greater detail below, passage of an expansion assembly therethrough causes radial expansion of sleeve body 12, typically to a final diameter of 5 mm, 10 mm, or 12 mm. Radially expandable sleeve 10 may be constructed in accordance with the details set forth in U.S. Pat. No. 5,431,676.

As seen in FIG. 2, distal end 12b of sleeve body 12 is flared radially outward. In particular, sleeve body 12 includes an intermediate portion 12c having a uniform diameter along substantially the entire length thereof, and a distal end 12b having a diameter which is larger than the diameter of intermediate portion 12c.

As seen in FIG. 1, a sheath 18 encases and/or otherwise covers sleeve body 12. Sheath 18 extends the entire length of sleeve body 12. Desirably, sheath 18 is fabricated from a plastic or elastomeric material, e.g., polyurethane, tetrafluorethylene, fluorinated ethylene-propylene, or the like. Desirably, sheath 18 will be weakened along an axial line (as by a pair of thin or weakened axial grooves or lines (not shown)) to facilitate splitting of sheath 18 at some point during the procedure. As described in more detail hereinafter, the axial grooves enable sheath 18 to be divided or split along the length thereof, as cannula assembly 40 is received in the lumen of sleeve body 12, and thus allow sleeve body 12 to radially expand.

Additionally, as seen in FIG. 1, sheath 18 helps to maintain flared distal end 12b closed (i.e., in a radially unexpanded condition) prior to introduction of the first surgical instrument. In other words, sheath 18 constricts distal end 12b such that distal end 12b has a diameter which is substantially equal to the diameter of intermediate portion 12c of sleeve body 12.

By way of example only, the braid of sleeve body 12 is preferably formed as a mesh of individual non-elastic filaments (e.g., composed of polyamide fiber, stainless steel, or the like) so that radial expansion causes axial shortening of the braid. Additionally, the braid of sleeve body 12 may be constructed from round filaments, flat or ribbon filaments, square filaments, or the like. Non-round filaments may advantageously reduce the axial force required to provide radial expansion. The filament width or diameter will typically be from about 0.002 inches (0.05mm) to about 0.25 inches (6.4mm), usually being from about 0.005 inches (0.1mm) to about 0.010 inches (0.3mm).

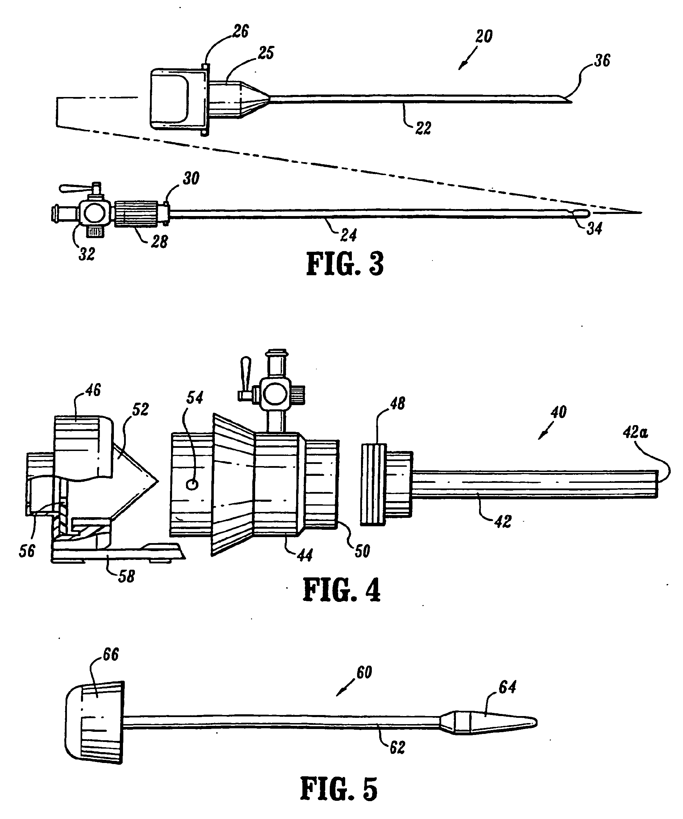

Turning now to FIG. 3, a pneumoperitoneum needle assembly, for use as part of an access system, is generally designated as 20. Pneumoperitoneum needle assembly 20 includes a tubular needle body 22, and a stylet 24 for operative engagement with tubular needle body 22. Tubular needle body 22 includes a hub 25, having a male bayonet connector 26 extending therefrom, provided at a proximal end thereof. Stylet 24 is spring-loaded in a connector 28 which is provided at a proximal end thereof Connector 28 includes a male bayonet fitting 30 which is receivably mounted in a female bayonet fitting (not illustrated) provided in hub 25 of needle body 22. Stylet 24 further includes an insufflation valve 32 provided at a proximal end thereof, and a port 34 formed in a distal end thereof Accordingly, insufflation gas, introduced through valve 32, is permitted to be released through port 34. In use, stylet 24 is to be mounted within tubular needle body 22 by way of bayonet fittings 30 of connector 28. The distal end of stylet 24 will extend from distal end 36 of needle body 22, and stylet 24 will retract into needle body 22 when needle body 22 is engaged against tissue, as described in more detail below.

Turning now to FIG. 4, a cannula assembly, for use as part of an access system, is generally designated as 40. Cannula assembly 40 includes a cannula tube 42, a cannula hub 44 connectable to cannula tube 42, and a valve cap 46 removably connectable to cannula hub 44. Cannula tube 42 includes a threaded connector 48 at a proximal end thereof which may be removably secured or connected to a fitting 50 provided at a distal end of cannula hub 44. Valve cap 46 desirably includes a pneumostasis valve element 52 and is configured to mate with a male bayonet fitting 54 provided at a proximal end of cannula hub 44. A second disk valve element 56 may be mounted in tandem with the pneumostasis valve element 52 to engage against an outer surface of a surgical instrument (not shown) when the surgical instrument is introduced through cannula assembly 40. Valve element 56 is generally sized for a relatively large instrument, e.g., an instrument having a diameter of about 12 mm. A reducing element 58 may be provided for reducing the size of the port of valve element 56 to accommodate relatively smaller instruments, e.g., instruments having a diameter of about 10 mm.

Turning now to FIG. 5, an obturator, for use as part of an access system, is generally designated as 60. Obturator 60 generally includes a shaft 62, a tapered distal end 64, and a handle 66. Obturator 60 is intended to be placed within a central lumen of cannula assembly 40 in order to form an expansion assembly for use as described below.

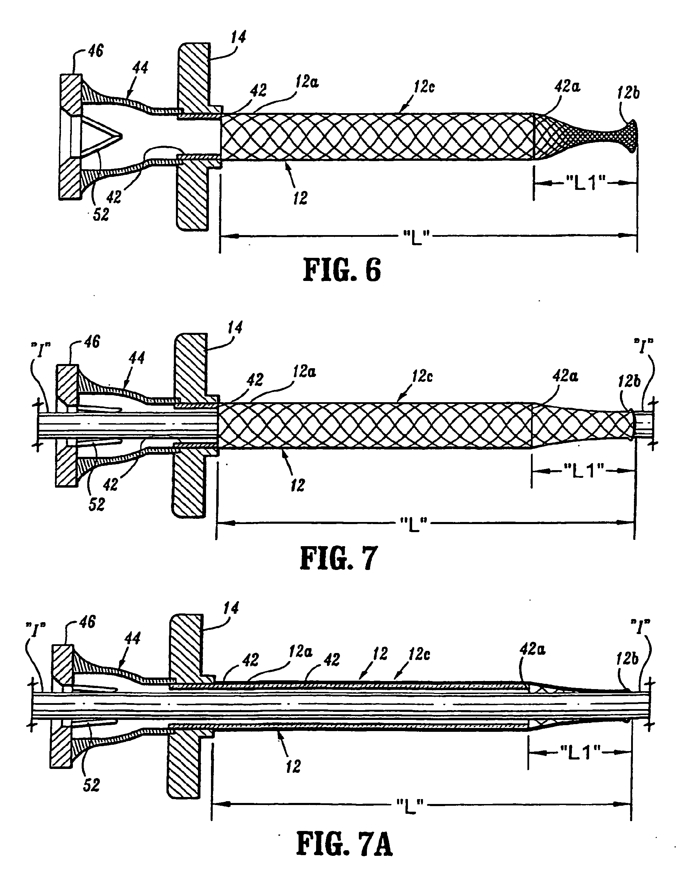

With reference now to FIG. 6, radially expandable sleeve component 10 is shown in operative association with cannula assembly 40. In particular, cannula tube 42 of cannula assembly 40 has been fully inserted into the lumen of sleeve body 12 of expandable sleeve component 10. Desirably, sleeve body 12 has a length "L" which is greater then the length of cannula tube 42 when cannula tube 42 has been fully inserted into expandable sleeve component 10. In this manner, distal end 12b of sleeve body 12 extends distally beyond a distal edge 42a of cannula tube 42. Desirably, length "L" of sleeve body 12 is such that flared distal end 12b thereof is spaced an axial distance "L1" from distal edge 42a of cannula tube 42 when cannula tube 42 is fully inserted into expandable sleeve component 10.

With reference to FIG. 7, flared distal end 12b of sleeve body 12 effectively forms and/or acts as an instrument seal against the surface of an instrument "I" introduced into and extending through cannula tube 42 of cannula assembly 40 and sleeve body 12 of expandable sleeve component 10. Flared distal end 12b of sleeve body 12 is provided in order to facilitate removal of instrument "I" from cannula assembly 40 and, in particular, from sleeve body 12 of expandable sleeve component 10.

Desirably, as seen in FIGS. 4, 6 and 7, pneumostasis valve element 52 of cannula hub 44 may take the form of a duck bill or "zero" valve. Valve element 52 may include two planar tapering portions which intersect at their distal ends to define an abutment face. The planar tapering portions may each include one or more inwardly directed, longitudinally oriented ribs to facilitate passage of instrument "I". The abutment face permits passage of instrument "I" through valve element 52, but in the absence of instrument "T", and particularly when cannula assembly 40 is inserted into an insufflated body cavity, the abutment face forms a gas-tight seal that isolates the insufflation cavity from the ambient surroundings. Valve element 52 also includes at least one, preferably two, reinforcing ribs (not shown) to stabilize valve element 52. The ribs are positioned to engage instrument "I" to guide instrument "T" through the slit of valve element 52 and prevent piercing of valve element 52 by the tip of instrument "I". Reference may be made to U.S Patent 5,603,702 for a more detailed discussion of a valve element.

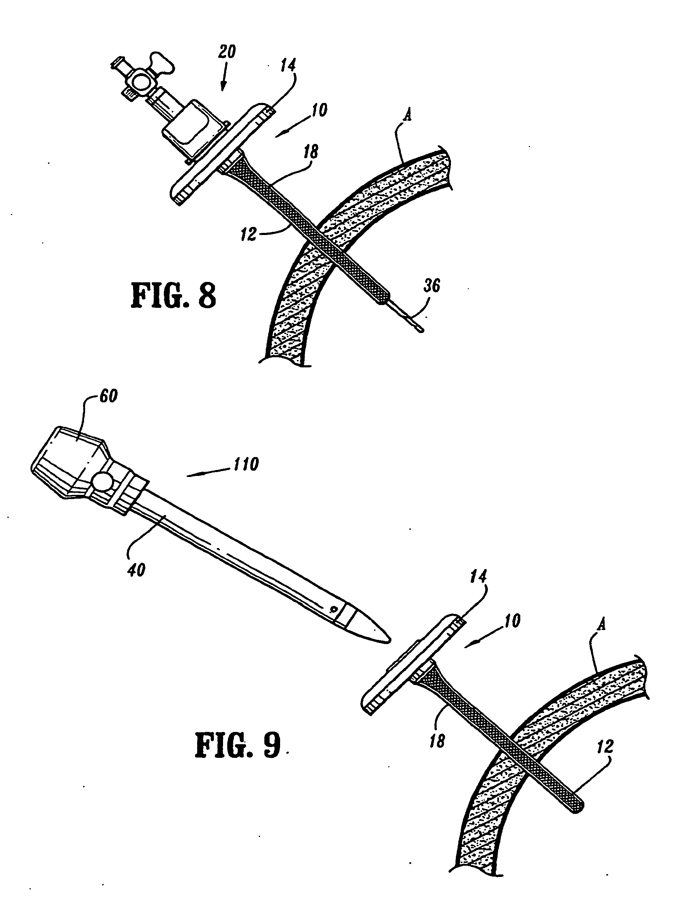

Referring now to FIGS. 8-13, use of radially expandable sleeve component 10, in an access system, will be described in detail. Initially, as seen in FIG. 8, a radially expandable sleeve component 10, having a pneumoperitoneum needle 20 inserted therein, is introduced through a patient's abdomen "A" (or other body location) by engaging sharpened distal end 36 of needle 20 against the tissue and advancing the assembly (e.g., expandable sleeve component 10 operatively coupled with needle 20) until sleeve body 12 of radially expandable sleeve component 10 extends across the tissue.

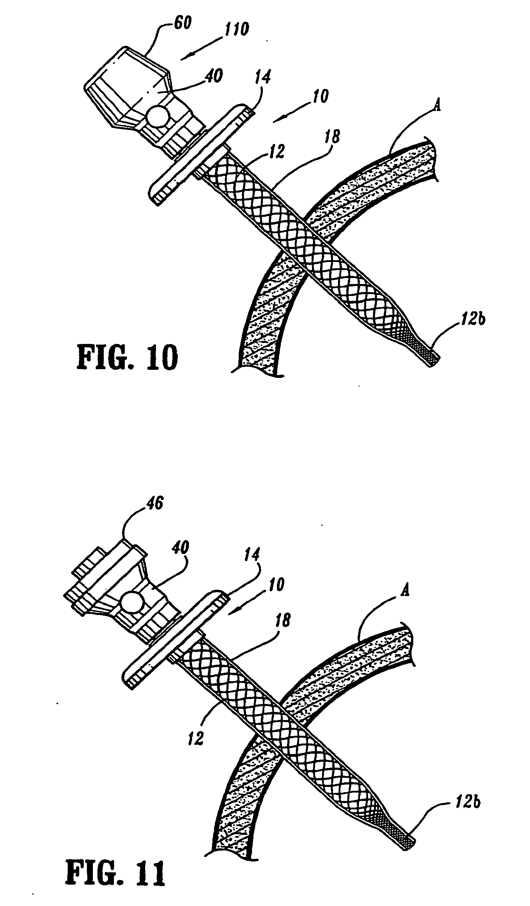

As seen in FIG. 9, needle 20 is removed from expandable sleeve component 10 and an expansion assembly 110, including cannula assembly 40 having obturator 60 operatively associated therewith, is introduced through radially expandable sleeve component 10. Introduction of expansion assembly 110 into radially expandable sleeve component 10 results in radial expansion of sleeve body 12 (see FIG. 10). In so doing, sheath 18 is divided or split along the length of the axial grooves (not shown). Additionally, insertion of expansion sleeve 110 into expandable sleeve component 10 to radially expand sleeve body 12 results in axial shortening of sleeve body 12 to thereby help anchor expansion assembly 110 in place and to help seal the exterior of expansion assembly 110 against the tissue.

As described above, when expansion assembly 110 is fully inserted into radially expandable sleeve component 10, distal edge 42a of cannula tube 42 does not extend beyond distal end 12b of sleeve body 12. Desirably, sleeve body 12 has a length "L" sufficient that when expansion assembly 110 is fully inserted into sleeve body 12 of expandable sleeve component 10, obturator 60 and cannula tube 40 do not radially expand distal end 12b of sleeve body 12 and, thus, do not split open a distal end of sheath 18.

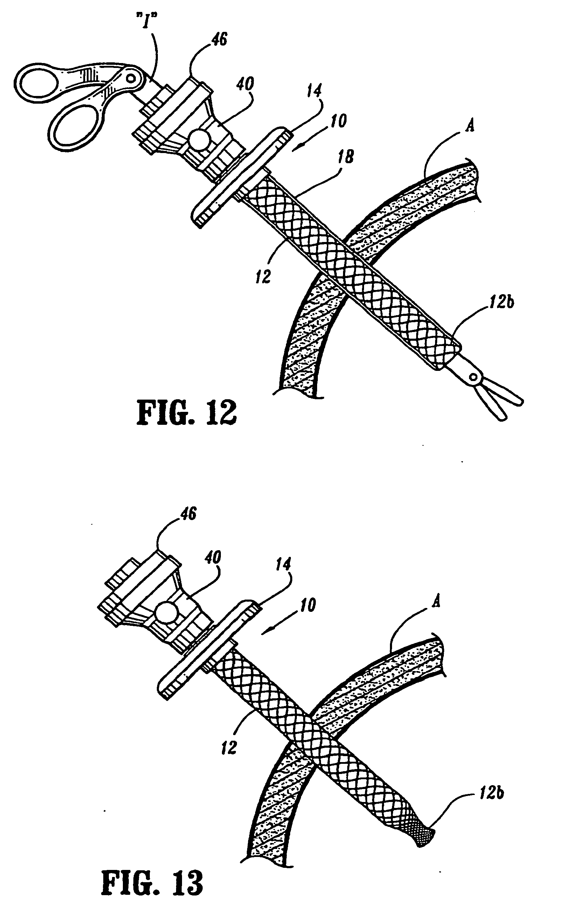

As seen in FIG. 11, obturator 60 may then be removed from cannula assembly 40 and radially expandable sleeve 40, leaving an access channel through abdominal wall "A". With obturator 60 removed, as seen in FIG. 12, a surgical instrument "I" (e.g., surgical graspers, staplers, tackers, fastener appliers, etc.) may be introduced, through cannula assembly 40 and radially expandable sleeve component 10, into the abdominal cavity. Desirably, instrument "I" has a length such that an end effector of instrument "I" is extendable beyond distal edge 42a of cannula tube 42 and beyond distal end 12b of sleeve body 12 of expandable sleeve component 10. Introduction of instrument "T" through distal end 12b of sleeve body 12 results in radial expansion of the same and, thus, in the dividing and/or splitting of the distal end of sheath 18. With sheath 18 divided along its entire length, it is now possible, if desired, to withdraw and remove sheath 18 from between the surface of the incision and sleeve body 12 of expandable sleeve component 10, as seen in FIG. 13.

With reference to FIG. 12, distal end 12b of sleeve body 12 acts as an instrument seal against the outer surface of instrument "I", thereby reducing the escape or passage of insufflation fluid through cannula tube 42. Such a fluid-tight seal is a particular advantage in laparoscopic procedures.

With reference to FIG. 13, following use of surgical instrument "I" in performing the surgical procedure, surgical instrument "I" may be removed and/or withdrawn from expansion assembly 110 and radially expendable sleeve component 10. Flared distal end 12b of sleeve body 12 facilitates the removal and/or withdrawal of surgical instrument "I" from expansion assembly 110 and radially expandable sleeve components 10. Additionally, flared distal end 12b of sleeve body 12 may act like a funnel to facilitate removal and/or retraction of a tissue or organ specimen from the abdominal cavity.

## Claims

1. An access system, comprising:
a radially expandable sleeve component (10), including:
a handle (14) having a passage therethrough; and
a sleeve body (12) having a proximal end (12a) connected to the handle, a distal end (12b), and an axial lumen (15) aligned with the passage of the handle, the sleeve body having a length; the access system further comprising:
a cannula tube (42) having a proximal end, a distal end, and a lumen extending therethrough, the cannula tube being sized to be received in the aperture of the handle of the radially expandable sleeve component, **characterised in that** the distal end of the sleeve body is flared radially outward, and the cannula tube has a length which is shorter than the length of the sleeve body when the cannula tube is fully inserted into the sleeve body of the radially expandable sleeve component, wherein when the cannula tube is fully inserted into the sleeve body of the radially expandable sleeve component the flared distal end of the sleeve body extends beyond the distal end of the cannula tube so that the flared distal end forms an instrument seal against the surface of an instrument introduced into and extending through the cannula tube and the sleeve body.

2. The access system according to any preceding claim, the sleeve body having a distal portion extending beyond the distal end of the cannula tube when the cannula tube is fully inserted into the sleeve body of the radially expandable sleeve component, wherein when the cannula tube is fully inserted into the sleeve body of the radially expandable sleeve component, the distal portion of the sleeve body tapers radially inwardly and at a distal end of the distal portion, the distal end of the sleeve body is flared radially outward so that when the cannula tube is fully inserted into the sleeve body of the radially expandable sleeve component, the inwardly tapered distal end portion of the sleeve body is able to engage the instrument (I) and seal against a surface of the instrument inserted into and extending through the cannula tube and the radially expandable sleeve component.

3. The access system according to claim 1 or 2, wherein the radially expandable sleeve further includes a sheath (18) encasing the sleeve body along at least a portion of the length thereof.

4. The access system according to claim 3, wherein the sheath maintains the flared distal end of sleeve body in a radially unexpanded condition.

5. The access system according claim 3 or 4, wherein the flared distal end of the sleeve body takes form upon removal of the sheath therefrom.

6. The access system of claim 3, 4 or 5, wherein the sheath includes a weakened axial line to allow the sheath to be split along the length thereof as the cannula tube is inserted therein, thus allowing the sleeve body to radially expand.

7. The access system of claim 6, comprising said instrument, the weakened line allowing a distal portion of the sleeve body extending beyond the distal end of the cannula tube to expand by the instrument splitting the distal end portion of sheath encasing the distal end portion of the sleeve body upon insertion thereof.

8. The access system of any one of the preceding claims, such that insertion of the cannula tube radially expands the sleeve body.

9. The access system of claim 8 as dependent on claim 3, 4, 5, 6 or 7, wherein the sleeve body has a distal portion extending beyond the distal end of the cannula tube when the cannula tube is fully inserted into the sleeve body of the radially expandable sleeve component, wherein the sheath maintains the distal portion in a radially unexpanded condition when the cannula tube is fully inserted into the sleeve body of the radially expandable sleeve component.

10. The access system according to any preceding claim, wherein the sleeve body is constructed from a radially expandable braid, which axially shortens the length of the sleeve body as the sleeve body is radially expanded.

11. The access system of any one of the preceding claims, wherein the flared distal end of the sleeve body facilitates withdrawal of instruments from the radially expandable sleeve component.

## Patentansprüche

1. Zugangssystem, umfassend:
eine radial aufweitbare Hülsenkomponente (10) mit:
einem Griff (14), der einen Durchgang aufweist; und
einem Hülsenkörper (12), der ein proximales Ende (12a), das mit dem Griff verbunden ist, ein distales Ende (12b) und einen axialen Hohlraum (15), der sich in einer Linie zum Durchgang des Griffes befindet, aufweist, wobei der Hülsenkörper eine Länge aufweist;
wobei das Zugangssystem ferner Folgendes umfasst:
ein Kanülenrohr (42), das ein proximales Ende, ein distales Ende und einen Hohlraum aufweist, der sich dort hindurch erstreckt, wobei das Kanülenrohr so bemessen ist, dass es in der Öffnung des Griffes der radial aufweitbaren Hülsenkomponente aufgenommen werden kann, **dadurch gekennzeichnet, dass** das distale Ende des Hülsenkörpers radial nach außen ausgestellt ist und das Kanülenrohr eine Länge aufweist, die kürzer ist als die Länge des Hülsenkörpers, wenn das Kanülenrohr vollständig in den Hülsenkörper der radial aufweitbaren Hülsenkomponente eingefügt ist, wobei, wenn das Kanülenrohr vollständig in den Hülsenkörper der radial aufweitbaren Hülsenkomponente eingefügt ist, sich das ausgestellte distale Ende des Hülsenkörpers über das distale Ende des Kanülenrohrs hinaus erstreckt, so dass das ausgestellte distale Ende eine Instrumentdichtung zur Oberfläche eines Instruments bildet, das in das Kanülenrohr und den Hülsenkörper eingeführt wird und sich dort hindurch erstreckt.

2. Zugangssystem nach einem der vorhergehenden Ansprüche, wobei der Hülsenkörper einen distalen Abschnitt aufweist, der sich über das distale Ende des Kanülenrohrs hinaus erstreckt, wenn das Kanülenrohr vollständig in den Hülsenkörper der radial aufweitbaren Hülsenkomponente eingefügt ist, wobei, wenn das Kanülenrohr vollständig in den Hülsenkörper der radial aufweitbaren Hülsenkomponente eingefügt ist, sich der distale Abschnitt des Hülsenkörpers radial nach innen verschmälert und an einem distalen Ende des distalen Abschnitts das distale Ende des Hülsenkörpers radial nach außen ausgestellt ist, so dass, wenn das Kanülenrohr vollständig in den Hülsenkörper der radial aufweitbaren Hülsenkomponente eingefügt ist, der radial nach innen verschmälerte distale Endabschnitt des Hülsenkörpers in der Lage ist, in das Instrument (I) einzugreifen und gegen eine Oberfläche des Instruments abzudichten, das in das Kanülenrohr und die radial aufweitbare Hülsenkomponente eingeführt wird und sich dort hindurch erstreckt.

3. Zugangssystem nach Anspruch 1 oder 2, wobei die radial aufweitbare Hülse ferner einen Mantel (18) umfasst, der den Hülsenkörper entlang von mindestens einem Abschnitt seiner Länge umhüllt.

4. Zugangssystem nach Anspruch 3, wobei der Mantel das ausgestellte distale Ende des Hülsenkörpers in einem radial nichtaufgeweiteten Zustand hält.

5. Zugangssystem nach Anspruch 3 oder 4, wobei das ausgestellte distale Ende des Hülsenkörpers nach Abnahme des Mantels seine Form annimmt.

6. Zugangssystem nach Anspruch 3, 4 oder 5, wobei der Mantel eine verdünnte axiale Linie umfasst, damit der Mantel entlang ihrer Länge gespaltet werden kann, wenn das Kanülenrohr eingefügt wird, wodurch sich der Hülsenkörper radial aufweiten kann.

7. Zugangssystem nach Anspruch 6, umfassend das Instrument, wobei es die verdünnte Linie einem distalen Abschnitt des Hülsenkörpers, der sich über das distale Ende des Kanülenrohrs hinaus erstreckt, ermöglicht, sich durch das Instrument aufzuweiten, das den distalen Endabschnitt des Mantels, der den distalen Endabschnitt des Hülsenkörpers umhüllt, nach seiner Einführung spaltet.

8. Zugangssystem nach einem der vorhergehenden Ansprüche, wobei das Einführen des Kanülenrohrs den Hülsenkörper radial aufweitet.

9. Zugangssystem nach Anspruch 8, wenn abhängig von Anspruch 3, 4, 5, 6 oder 7, wobei der Hülsenkörper einen distalen Abschnitt aufweist, der sich über das distale Ende des Kanülenrohrs hinaus erstreckt, wenn das Kanülenrohr vollständig in den Hülsenkörper der radial aufweitbaren Hülsenkomponente eingefügt ist, wobei der Mantel den distalen Abschnitt in einem radial nichtaufgeweiteten Zustand hält, wenn das Kanülenrohr vollständig in den Hülsenkörper der radial aufweitbaren Hülsenkomponente eingefügt ist.

10. Zugangssystem nach einem der vorhergehenden Ansprüche, wobei der Hülsenkörper aus einem radial aufweitbaren Geflecht konstruiert ist, das die Länge des Hülsenkörpers axial verkürzt, wenn der Hülsenkörper radial aufgeweitet wird.

11. Zugangssystem nach einem der vorhergehenden Ansprüche, wobei das ausgestellte distale Ende des Hülsenkörpers die Entnahme von Instrumenten aus der radial aufweitbaren Hülsenkomponente erleichtert.

## Revendications

1. Système d'accès, comprenant :
un composant de manchon radialement expansible (10), contenant :
un manche (14) doté d'un passage le traversant ; et
un corps de manchon (12) présentant une extrémité proximale (12a) reliée au manche, une extrémité distale (12b) et un lumen axial (15) aligné avec le passage du manche, le corps de manchon présentant une longueur ; le système d'accès comprenant en outre :
un tube de canule (42) présentant une extrémité proximale, une extrémité distale, et un lumen le traversant, le tube de canule étant dimensionné de sorte à être reçu dans l'ouverture du manche du composant de manchon radialement expansible, **caractérisé en ce que** l'extrémité distale du corps de manchon est évasée radialement vers l'extérieur, et le tube de canule présente une longueur plus courte que la longueur du corps de manchon lorsque le tube de canule est pleinement inséré dans le corps de manchon du composant de manchon radialement expansible, dans lequel lorsque le tube de canule est pleinement inséré dans le corps de manchon du composant de manchon radialement expansible, l'extrémité distale évasée du corps de manchon s'étend au-delà de l'extrémité distale du tube de canule de sorte que l'extrémité distale évasée forme une garniture d'instrument contre la surface d'un instrument introduit dans et s'étendant au travers du tube de canule et du corps de manchon.

2. Système d'accès selon l'une quelconque des revendications précédentes, le corps de manchon présentant une partie distale s'étendant au-delà de l'extrémité distale du tube de canule lorsque le tube de canule est pleinement inséré dans le corps de manchon du composant de manchon radialement expansible, dans lequel lorsque le tube de canule est pleinement inséré dans le corps de manchon du composant de manchon radialement expansible, la partie distale du corps de manchon se réduit radialement vers l'intérieur et sur une extrémité distale de la partie distale, l'extrémité distale du corps de manchon est évasée radialement vers l'extérieur de sorte que lorsque le tube de canule est pleinement inséré dans le corps de manchon du composant de manchon radialement expansible, la partie d'extrémité distale réduite vers l'intérieur du corps de manchon est capable d'engager l'instrument (I) et de rendre étanche contre une surface de l'instrument inséré dans et s'étendant au travers du tube de canule et du composant de manchon radialement expansible.

3. Système d'accès selon la revendication 1 ou 2, dans lequel le manchon radialement expansible contient en outre une gaine (18) enrobant le corps de manchon le long d'au moins une partie de sa longueur.

4. Système d'accès selon la revendication 3, dans lequel la gaine maintient l'extrémité distale évasée du corps de manchon dans un état radialement non dilaté.

5. Système d'accès selon la revendication 3 ou 4, dans lequel l'extrémité distale évasée du corps de manchon prend forme suite au retrait de la gaine.

6. Système d'accès selon la revendication 3, 4 ou 5, dans lequel la gaine contient une ligne axiale affaiblie pour permettre à la gaine d'être fendue le long de sa longueur lorsque le tube de canule est inséré dedans, permettant ainsi au corps de manchon de se dilater radialement.

7. Système d'accès selon la revendication 6, comprenant ledit instrument, la ligne affaiblie permettant à une partie distale du corps de manchon s'étendant au-delà de l'extrémité distale du tube de canule de se dilater par l'instrument fendant la partie d'extrémité distale de la gaine enrobant la partie d'extrémité distale du corps de manchon suite à son insertion.

8. Système d'accès selon l'une quelconque des revendications précédentes tel que l'insertion du tube de canule dilate radialement le corps de manchon.

9. Système d'accès selon la revendication 8, si elle dépend de la revendication 3, 4, 5, 6, ou 7, dans lequel le corps de manchon présente une partie distale s'étendant au-delà de l'extrémité distale du tube de canule lorsque le tube de canule est pleinement inséré dans le corps de manchon du composant de manchon radialement expansible, dans lequel la gaine maintient la partie distale dans un état radialement non dilaté lorsque le tube de canule est pleinement inséré dans le corps de manchon du composant de manchon radialement expansible.

10. Système d'accès selon l'une quelconque des revendications précédentes, dans lequel le corps de manchon est construit à partir d'un galon radialement expansible, qui raccourcit axialement la longueur du corps de manchon lorsqu'il est radialement dilaté.

11. Système d'accès selon l'une quelconque des revendications précédentes, dans lequel l'extrémité distale évasée du corps de manchon facilite le retrait des instruments du composant de manchon radialement expansible.
